# EUROPEAN PATENT SPECIFICATION

(11) **EP 1 794 268 B1**
(45) Date of publication and mention of the grant of the patent: **28.07.2010**
(21) Application number: 05761042.0
(22) Date of filing: 15.06.2005
(51) Int. Cl.: C11D 1/72, C11D 3/37, C11D 3/48, C11D 3/02, C11D 3/06, C11D 3/20, C11D 3/22, A61L 2/18

(54) **Use of solutions for ophthalmic lenses containing at least one silicone containing component**
Verwendung von Lösungen für Kontaktlinsen mit mindestens einer Silikonhaltigen Komponente
Utilisation des solutions pour lentilles ophtalmiques contenant au moins un composant renfermant un silicone

(30) Priority: 30.06.2004 US 880941
(43) Date of publication of application: 13.06.2007
(73) Proprietor: Johnson and Johnson Vision Care, Inc., Jacksonville, FL 32256 (US)
(72) Inventor: RATHORE, Osman, Jacksonville, FL 32256 (US); PALL, Brian, Jacksonville, FL 32258 (US); MEYERS, Ann-Marie, Wong, Jacksonville, FL 32216 (US); BROWN-SKROBOT, Susan, Jacksonville, FL 32256 (US); NEELY, Frank, Jacksonville, FL 32259 (US); ALVAREZ-CARRIGAN, Nayiby, St. Augustine, FL 32092 (US)
(74) Representative: Fisher, Adrian John
(86) International application number: PCT/US2005/021076
(87) International publication number: WO 2006/011999

(56) References cited:
- EP-A- 0 591 956
- EP-A- 0 888 780
- WO-A-00/37048
- WO-A-01/23509
- WO-A-95/00617
- WO-A-02/087326
- WO-A-2004/014443
- WO-A-2004/045661
- WO-A-2004/047879
- US-A- 3 882 036
- US-A- 5 387 394
- US-A- 5 711 823
- US-A- 6 037 328
- US-A1- 2001 001 789
- US-A1- 2002 004 466
- US-A1- 2003 130 144
- US-A1- 2003 153 475
- US-A1- 2003 190 258
- US-A1- 2004 063 591
- US-A1- 2004 120 916
- US-B1- 6 617 291

## Description

### Field of the Invention

The present invention relates to solutions for use with ophthalmic lenses. More particularly, the present invention relates to solutions which are suitable for use with uncoated silicone hydrogel contact lenses.

### Background of the Invention

Soft contact lenses have been commercially available since the mid 1980s. Recently, soft contact lenses displaying improved oxygen permeability have been introduced. Focus N&D (by Ciba Vision) and Purevision (by Bausch and Lomb) have high oxygen permeabilities compared to conventional hydrogels, but require specialized treatment in manufacture to impart surface wettability. However, the surface treatment processes add cost and complexity to the lens manufacturing process. Accordingly, silicone hydrogel lenses which do not need surface modification are desirable and have recently been introduced.

Contact lenses having antimicrobial components, such as antimicrobial metal salts, have also been disclosed. Contact lens solutions which are compatible with these lenses are desired.

US2004063591; WO0123509, US5711823, WO0037048, WO02087326, US6037328, US2002004466 and US2001001789 all describe ophthalmic solutions for use with contact lenses containing silicone, wherein the solution has an osmolality of 220 mOsm/kg or greater.

### Summary of the Invention

The present invention relates to the use of ophthalmic solutions for contacting an uncoated ophthalmic lens comprising polymer units derived from at least one silicone containing component, to reduce the occurrence of superficial punctate staining on the cornea resulting from wearing said lens, wherein said solution comprises an osmolality of 220 mOsm/kg or greater, and a conductivity of 4mS/cm or greater.

The present invention further comprises a method for reducing the occurrence of superficial punctate staining comprising the step of contacting an uncoated ophthalmic lens comprising polymer units derived from at least one silicone containing component in a solution comprising an osmolality of about 220 mOsm/kg or greater, and a conductivity of 4mS/cm or greater.

### Detailed Description of the Specific Embodiments

As used herein, the terms "contact lens" and "ophthalmic device" refer to devices that reside in or on the eye. These devices can provide optical correction, wound care, drug delivery, diagnostic functionality, cosmetic enhancement or effect or a combination of these properties. The term lens (or contact lens) includes but is not limited to soft contact lenses, hard contact lenses, intraocular lenses, overlay lenses, ocular inserts, and optical inserts.

All percentages in this specification are weight percentages unless otherwise noted.

As used herein, the phrase "without a surface treatment" or "not surface treated" means that the exterior surfaces of the devices of the present invention are not separately treated to improve the wettability of the device. Treatments which may be foregone because of the present invention include, plasma treatments, grafting, coating and the like. However, coatings which provide properties other than improved wettability, such as, but not limited to antimicrobial coatings and the application of color or other cosmetic enhancement, may be applied to devices of the present invention.

It has been surprisingly found that when a contact lens comprising at least one silicone containing component is contacted or stored in a solution having a conductivity of 4mS/cm or greater and an osmolality of 220 mOsm/kg or greater, and preferably an osmolality of 230 mOsm/kg or greater the incidence of superficial punctate staining on the cornea of the eye resulting from contact lens wear is dramatically reduced or eliminated.

The solution may be any water-based solution that is used for the packaging, storage and/or cleaning of contact lenses, so long as the osmolality meets the requirements specified herein. Typical solutions include, without limitation, saline solutions, buffered solutions, buffered saline solutions and deionized water. The preferred aqueous solution is saline solution containing salts including, without limitation, sodium chloride, sodium sulfate, sodium acetate, sodium citrate, sodium borate, sodium phosphate, sodium hydrogenphosphate, sodium dihydrogenphosphate, sodium lactate or the corresponding potassium, calcium or magnesium salts of the same and the like. Non-ionic compounds may also be used to provide the desired osmolality. Suitable non-ionic compounds include polyethylene glycols, polyhydroxy compounds, polyether compounds, sugars, polyvinylamides (such as PVP, PVMA), dextrans, cyclodextrans and mixtures thereof and the like. Combinations of ionic and non-ionic compounds may also be used. These ingredients are generally combined to form buffered solutions that include an acid and its conjugate base, so that addition of acids and bases cause only a relatively small change in pH.

The solutions may additionally include 2-(N-morpholino)ethanesulfonic acid (MES), sodium hydroxide, 2,2-bis(hydroxymethyl)-2,2',2"-nitrilotriethanol, n-tris(hydroxymethyl)methyl-2-aminoethanesulfonic acid, citric acid, sodium citrate, sodium carbonate, sodium bicarbonate, acetic acid, sodium acetate, and the like and combinations thereof. Preferably, the solution is a borate buffered or phosphate buffered saline solution or borate buffered sodium sulfate solution.

Osmolality is a measure of the number of particles present in solution and is independent of the size or weight of the particles. It can be measured only by use of a property of the solution that is dependent only on the particle concentration. These properties are collectively referred to as Colligative Properties (vapor pressure depression, freezing point depression, boiling point elevation, osmotic pressure). Osmolality of a solution is the number of osmoles of solute per kilogram of solvent. This is the amount of a substance that yields, in ideal solution, that number of particles (Avogadro's number) that would depress the freezing point of the solvent by 1.86K. The osmolality values reported in the Examples were measured via freezing point depression using a Micro-Osmometer Model 3 MOplus. Solutions having the osmalility specified herein may be readily prepared by incorporating appropriate amounts of ionic salts, such as those listed above. A suitable concentration range for the salt(s) are between 0.01 to 5 weight% and preferably between 0.1 to 3.0 weight % as part of a buffer system (such as borate or phosphate). An example of a suitable packaging solution includes 0.2 weight % sodium borate, 0.9 weight % boric acid, 0.2 weight % chloride from sodium chloride. Other solutions with differing amounts of components will be readily apparent to those of skill in the art and are useful in the present invention.

Conductivity is the ability of a material to conduct electric current. Conductivity may be measured using commercial conductivity probes. Solutions having the desired conductivities may be made by incorporating the salts listed above. Salts which are more conductive, such as, for example sodium chloride, may be used in lesser quantities than salts with relatively low conductivities such as sodium borate.

In a further embodiment solutions used in the present invention comprise an osmolality of at least 220 mOsm/kg and a conductivity of at least 5 mS/cm., or an osmolality of at least 230 mOsm/kg and a conductivity of at least 4 mS/cm., and most preferably an osmolality of at least 230 mOsm/kg and a conductivity of at least 5 mS/cm.

The solutions used in the present invention may also comprise any known active and carrier components useful for lens packaging solution. Suitable active ingredients for lens packaging solutions include, without limitation, antibacterial agents, anti-dryness agents, such as polyvinyl alcohol, polyvinylpyrrolidone, dextran, polyethylene oxides, hydroxy propylmethyl cellulose (HPMC), tonicity agents, pharmaceuticals, nutraceuticals, additives which prevent the lens from sticking to the package and the like, and combinations thereof. To form the solution, the ingredients are combined with the water-based solution, stirred, and dissolved. The pH of the solution preferably is adjusted to 6.2 to 7.7. The lens to be stored in the packaging solution of the invention is immersed in the solution and the solution and lens placed in the package in which the lens is to be stored. Alternatively, the solution may be placed into the package and the lens then placed into the solution. Typically, the package is then sealed by any convenient method, such as by heat sealing, and undergoes a suitable sterilization procedure.

The lenses which may be beneficially contacted or immersed in the solutions in accordance with the present invention comprise polymeric units derived from at least one silicone containing component.

Silicone containing components contain at least one [—Si—O—Si] group, in a monomer, macromer or prepolymer. Preferably, the Si and attached O are present in the silicone containing component in an amount greater than 20 weight percent, and more preferably greater than 30 weight percent of the total molecular weight of the silicone containing component. Useful silicone containing components preferably comprise polymerizable functional groups such as acrylate, methacrylate, acrylamide, methacrylamide, N-vinyl lactam, N-vinylamide, vinylcarbonate, vinylcarbamate, and styryl functional groups. Examples of silicone containing monomers which are useful in this invention include 3-methacryloxypropyltris(trimethylsiloxy)silane (TRIS), amide analogs of TRIS described in U.S. 4,711,943, vinylcarbamate or carbonate analogs decribed in U.S. Pat. 5,070,215, siloxane containing monomers contained in U.S. Pat. 6,020,445, difunctional substituted and unsubstituted polysiloxanes described in US 2002/0016383 and JP2002-327063, are useful.

More specifically, silicone-containing monomers include 3-methacryloxypropyltris(trimethylsiloxy)silane (TRIS), monomethacryloxypropyl terminated polydimethylsiloxanes, polydimethylsiloxanes, 3-methacryloxypropylbis(trimethylsiloxy)methylsilane, methacryloxypropylpentamethyl disiloxane, polysiloxanedimethacrlyate having alcohol or terminal methoxy type polyoxyethylene groups, such as shown in Synthesis Examples 1- 6 of US 2002/0016383 and combinations thereof.

Suitable silicone containing macromers which are useful in the present invention have a number average molecular weight between about 5,000 and about 15,000 Daltons. Silicone containing macromers include materials comprising at least one siloxane group, and preferably at least one dialkyl siloxane group and more preferably at least one dimethyl siloxane group. The silicone containing macromers may include other components such as urethane groups, alkylene or alkylene oxide groups, polyoxyalkalene groups, arylene groups, alkyl esters, amide groups, carbamate groups, perfluoroalkoxy groups, isocyanate groups, combinations thereof of and the like. Silicone containing macromers may be formed via group transfer polymerization ("GTP"), free radical polymerization, condensation reactions and the like. Specific silicone containing macromers, and methods for their manufucture, include those disclosed in US 5,760,100 as materials A-D (methacrylate functionalized, silicone-fluoroether urethanes and methacrylate functionalized, silicone urethanes), and those disclosed in US 6,367,929 (styrene functionalized prepolymers of hydroxyl functional methacrylates and silicone methacrylates), difunctional organosiloxane macromers containing polyoxyalkylene units as disclosed in JP 2001-183502 and JP 2001-188101.

Suitable silicone containing prepolymers include vinyl carbamate functionalized polydimethylsiloxane, which is further disclosed in US 5,070215 and urethane based prepolymers comprising alternating "hard" segments formed from the reaction of short chained diols and diisocyantes and "soft" segments formed from a relatively high molecular weight polymer, which is α,ω endcapped with two active hydrogens. Specific examples of suitable silicone containing prepolymers, and methods for their manufacture, are disclosed in US 5,034,461.

In one embodiment, the lens further comprises at least one polymeric internal wetting agent. As used herein, "polymeric wetting agent" refers to substances having a weight average molecular weight of at least 2,500 Daltons. The preferred weight average molecular weight of these polymeric wetting agents is greater than 100,000; more preferably between 150,000 to 2,000,000 Daltons, more preferably still between 300,000 to 1,800,000 Daltons.

Alternatively, the molecular weight of polymeric wetting agents can be also expressed by the K-value, based on kinematic viscosity measurements, as described in Encyclopedia of Polymer Science and Engineering, N-Vinyl Amide Polymers, Second edition, Vol 17, pgs. 198-257, John Wiley & Sons Inc. When expressed in this manner, hydrophilic monomers having K-values of greater than 12 and preferably between 30 and 150.

The way in which the polymeric wetting agent is added to the lens is not critical. The polymeric wetting agent may be added to the reaction mixture as a polymer, may be formed from at least one hydrophilic monomer which is added to the reaction mixture and forms a hydrophilic polymer upon curing of the reaction mixture or may be added after the lens is formed in the packaging solution. Examples of polymeric wetting agents include but are not limited to polymers and copolymers comprising polyamides, polylactones, polyimides, polylactams, polyacrylic acid and functionalized polyamides, polylactones, polyimides, polyacrylic acid, polylactams, such as DMA functionalized by copolymerizing DMA with a lesser molar amount of a hydroxyl-functional monomer such as HEMA, and then reacting the hydroxyl groups of the resulting copolymer with materials containing radical polymerizable groups, such as isocyanatoethylmethacrylate or methacryloyl chloride. Hydrophilic prepolymers made from DMA or n-vinyl pyrrolidone with glycidyl methacrylate may also be used. The glycidyl methacrylate ring can be opened to give a diol which may be used in conjunction with other hydrophilic prepolymer in a mixed system to increase the compatibility of the high molecular weight hydrophilic polymer, hydroxyl-functionalized silicone containing monomer and any other groups which impart compatibility. Polymeric wetting agents include but are not limited to those disclosed in US US 6,367,929, WO 2003/022321 and acyclic polyamides comprising repeating units of Formula I

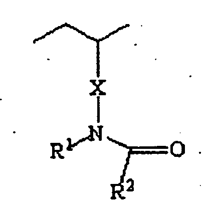

Wherein X is a direct bond, 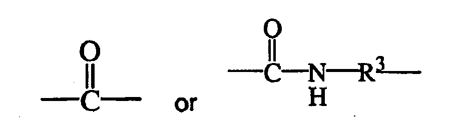 wherein R³ is a C1 to C3 alkyl group;
R¹ is selected from H, straight or branched, substituted or unsubstituted C1 to C4 alkyl groups,
R² is selected from H, straight or branched, substituted or unsubstituted C1 to C4 alkyl groups, amino groups having up to two carbons, amide groups having up to 4 carbon atoms and alkoxy groups having up to two carbons and wherein the number of carbon atoms in R¹ and R² taken together is 8, preferably 6 or less.

Homopolymers and copolymers comprising N-vinylpyrrolidone, N-vinyl-N-methylacetamide, (meth)acrylic acid, N, N-dimethylacrylamide, combinations thereof and the like are particularly preferred.

In addition to the polymeric units derived from silicone containing components and the polymeric wetting agent, the lens may also include polymeric units derived from hydrophilic components, compatibilizing agents such as those disclosed in WO 2003/022321, WO 2002/022322 and US 10/794,399, hydroxyl containing components, fluorine containing components, cross-linkers, photoinitiators, UV absorbers, medicinal agents, antimicrobial compounds, reactive tints, pigments, copolymerizable and nonpolymerizable dyes, release agents, combinations thereof and the like. These additional components may be incorporated into the lens in any way, such as, but not limited to polymerized into the lens matrix, mixed into the monomer mix used to make the lens or absorbed into the lens. One class of lens additives which may be included are antimicrobial metals, including but not limited to antimicrobial metal salts, such as those disclosed in US 10/715,903.

Suitable hydrophilic components are well known in the art and are disclosed in WO 2003/022321. Preferred hydrophilic monomers which may be incorporated into the polymer of the present invention include hydrophilic monomers such as N,N-dimethyl acrylamide (DMA), 2-hydroxyethyl acrylate, glycerol methacrylate, 2-hydroxyethyl methacrylamide, N-vinylpyrrolidone (NVP), HEMA, and polyethyleneglycol monomethacrylate. Most preferred hydrophilic monomers include DMA, NVP, HEMA and mixtures thereof.

In certain embodiments a hydroxyl containing component may also be included. The hydroxyl containing component that may be used to make the polymers of this invention have at least one polymerizable double bond and at least one hydrophilic functional group. Examples of polymerizable double bonds include acrylic, methacrylic, acrylamido, methacrylamido, fumaric, maleic, styryl, isopropenylphenyl, O-vinylcarbonate, O-vinylcarbamate, allylic, O-vinylacetyl and N-vinyllactam and N-vinylamido double bonds. The hydroxyl containing component may also act as a crosslinking agent. In addition the hydroxyl containing component comprises a hydroxyl group. This hydroxyl group may be a primary, secondary or tertiary alcohol group, and may be located on an alkyl or aryl group. Examples of hydroxyl containing monomers that may be used include but are not limited to 2-hydroxyethyl methacrylate ("HEMA"), 2-hydroxyethyl acrylate, 2-hydroxyethyl methacrylamide, 2-hydroxyethyl acrylamide, N-2-hydroxyethyl vinyl carbamate, 2-hydroxyethyl vinyl carbonate, 2-hydroxypropyl methacrylate, hydroxyhexyl methacrylate, hydroxyoctyl methacrylate and other hydroxyl functional monomers as disclosed in U.S. Patents 5,006,622; 5,070,215; 5,256,751 and 5,311,223. Preferred hydrophilic components include 2-hydroxyethyl methacrylate.

Lenses of the present invention may be formed via known methods such as mixing the components which are polymerized or entangled to form the lens (the reactive components) either alone or with a diluent(s) and a polymerization initiator and curing by appropriate conditions to form a product. Various processes are known for processing the reaction mixture in the production of contact lenses, including spincasting and static casting. Spincasting methods are disclosed in U.S. Pat. Nos. 3,408,429 and 3,660,545, and static casting methods are disclosed in U.S. Pat. Nos. 4,113,224 and 4,197,266, 4,495,313; 4,680,336; 4,889,664; and 5,039,459.

Suitable ranges of the various components (in weight % based upon all reactive components) are shown in Table 1, below.

| | Wt % | | |
|---|---|---|---|
| Silicone containing component | 5-95 | 30-85 | 45-75 |
| Polymeric wetting agent | 1-15 | 3-15 | 3-12 |
| Hydrophilic component | 5-80 | 10-60 | 20-50 |

The weight percents above are based upon all reactive components. Thus, the lenses may have any of the compositional ranges listed in the table, which describes twenty-seven possible compositional ranges. Each of the ranges listed above is prefaced by the word "about". The foregoing range combinations are presented with the proviso that the listed components, and any additional components add up to 100 weight%.

Specific examples of lens materials containing both a silicone containing component and a polymeric wetting agent include compositions comprising (a) at least one silicone containing component selected from the group consisting of monomethacryloxypropyl terminated polydimethylsiloxanes, vinyl carbamate functionalized polydimethylsiloxane, methacrylate functionalized, silicone-fluoroether urethanes, methacrylate functionalized silicone urethanes, styrene functionalized prepolymers of hydroxyl functional methacrylates and silicone methacrylates, difunctional organosiloxane macromers containing polyoxyalkylene units, urethane based prepolymers hydrophilic siloxane containing monomers and macromers which may be fluorine substituted and (b) at least one polymeric wetting agent selected from homopolymers and/or copolymers comprising repeating units from N-vinylpyrrolidone, N,N-dimethacylamide, N-vinyl-N-methylacetamide. Examples of specific formulations include, but are not limited to galyfilcon and senofilcon.

According to the present invention lenses comprising at least one polymeric wetting agent and polymeric units derived from at least one silicone containing component are contacted with a solution having a specified osmolality. "Contacted" includes immersing the lens in the solution, such as during packaging by the manufacturer or storage by the consumer as well as spraying, dipping or washing the lens such as during cleaning.

In order to illustrate the invention the following examples are included. These examples do not limit the invention. They are meant only to suggest a method of practicing the invention. Those knowledgeable in contact lenses as well as other specialties may find other methods of practicing the invention. However, those methods are deemed to be within the scope of this invention.

The following test methods were used in the Examples.

Osmolality was measured using a Micro-Osmometer Model 3 MOplus and the following procedure. The instrument was internally calibrated with MST traceable 50 mOsm and 850 mOsm standards. The solutions to be tested were kept sealed in a vial until evaluation. The sampling system (a pipette fitted with a plunger) was rinsed by pipetting sample solution into the barrel of the sampling system and discarding. Solution was pipetted into the sample system, the sample system was placed in the osmometer and the osmolality was measured. The measurement was repeated three times and the average is reported.

Conductivity is measured using a FISHER^{®} ACCUMET^{®} 150 and the following procedure. The instrument is calibrated using NIST traceable conductivity standards. The solutions to be tested were kept sealed in a vial until evaluation. About 30 ml of solution was placed in a hinged cap sample vial. The conductivity probe is dipped into the solution at least three times prior to sample evaluation to wet the probe and remove any bubbles. The conductivity probe and automatic temperature compensation probe are immersed in the sample solution and the conductivity is recorded when the reading on the instrument stabilizes.

### Examples

### Example 1

100 parts of the components shown in Table 2 and, in the amounts shown in Table 2 were mixed with 23 parts 3,7-dimethyl-3-octanol (D₃O) to form a reaction mixture. The reaction mixture was mixed vigorously (or until the solution appeared clear and evenly mixed) and then degassed, on high vacuum.

**Table 2**

| **Component** | **Weight Per Cent** |
|---|---|
| Norbloc | 2.00 |
| CGI 1850 | 0.48 |
| mPDMS 1000 | 31.00 |
| DMA | 24.00 |
| HEMA | 6.00 |
| TEGDMA | 1.50 |
| SiMAA2 | 28.00 |
| Blue HEMA | 0.02 |
| K90 | 7.00 |

The reaction mixture was then placed into thermoplastic contact lens molds and irradiated using fluorescent bulbs (intensity of about 1 mW/cm² for 8 minutes and about 4 mW/cm² for 4 minutes) at 45 °C under a nitrogen atmosphere. After curing, the molds were opened, the lenses were demolded and hydrated as follows 70/30 IPA:DI water for 60 min, 100% IPA for 60 min, 70/30 IPA:DI water for 60 min, 10/90 IPA:DI water for 30 min and equilibrate in DI water. The hydrated lenses were stored in jars containing DI-water with 50 ppm of methylcellulose.

### Examples 2-6

To a borate buffer solution (0.185 wt% sodium borate, 0.926% boric acid and 0.005wt% methyl cellulose) sodium chloride was added in varying amounts, as shown in Table 3, below. The osmolality and conductivity for each solution is also listed. Lenses made in Examples 1 were placed in the solutions listed in Table 3, below. The lenses were autoclaved once in the respective packaging solutions. Lenses were removed from the package and immediately applied to each subject's eye. The lenses were intentionally not rinsed prior to insertion.

Subjects wore the lenses for approximately 30 minutes prior to lens evaluation. Lenses were then removed using standard clinical techniques.

After lenses were removed, corneal epithelial integrity was evaluated using fluorescein sodium dye. Fluorescein sodium dye strips were moistened using a couple of drops of non-preserved saline and the strip was lightly dabbed on the bulbar conjunctiva of each eye. After a brief period (5-10 seconds), each cornea was evaluated using both a cobalt blue and yellow Wratten filter to accentuate the appearance of the dye. Any break in the corneal epithelial integrity, or corneal staining, when noted, was graded using a 1 to 4 grading scale. If any corneal staining was noted, that patient was included as displaying corneal staining in the percentages listed in Table 3, below.

Upon completion of the procedure, the residual fluorescein sodium dye was rinsed out using non-preserved saline.

**Table 3**

| Ex. # | [Cl-] wt% | Conductivity (mS/cm) | Osmolaltiy (mOsm/kg) | # eyes | % staining |
|---|---|---|---|---|---|
| 2* | 0 | 0.7 | 163 | 6 | 100 |
| 3* | 0.086 | 3.1 | 207 | 8 | 100 |
| 4* | 0.12 | 4.0 | 217 | 12 | 50 |
| 5 | 0.16 | 5.0 | 234 | 10 | 0 |
| 6 | 0.22 | 6.8 | 273 | 6 | 0 |

| | | | | | |
|---|---|---|---|---|---|
| *comparative | | | | | |

### Examples 7-10

Examples 2-6 were repeated except that sodium sulfate was used instead of sodium chloride. The results are shown in Table 4, below.

**Table 4**

| Ex. # | [Cl-] wt% | Conductivity (mS/cm) | Osmolaltiy (mOsm/kg) | # eyes | % staining |
|---|---|---|---|---|---|
| 7* | 0 | 0.7 | 163 | 6 | 100 |
| 8 | 0.37 | 5.1 | 223 | 10 | 30 |
| 9 | 0.48 | 6.4 | 245 | 10 | 0 |
| 10 | 1.4 | 15.3 | 382 | 6 | 0 |

| | | | | | |
|---|---|---|---|---|---|
| *comparative | | | | | |

### Example 11 (comparative)

The procedure of Examples 2-6 was repeated, except that PEG400 (commercially available from Aldrich), was used in a concentration of 3.15 weight %. The resulting solution has a conductivity of 0.712 mS/cm and an osmolality of 261 mS/cm. Ten lenses were stored and evaluated as described in Examples 2-6. Superficial punctuate staining was observed in two of the ten eyes which were screened.

### Example 12

The procedure of Examples 2-6 was repeated, except that sodium lactate, was used. Sodium lactate packaging solution was prepared by dissolving 34.2 +/-0.2 g sodium lactate in 4L borate buffer (0.185 wt% sodium borate, 0.926% boric acid) containing 50 ppm methylcellulose. The resulting solution has a conductivity of 5.92+/-0.005 mS/cm and an osmolality of 283+/-0.5 mOsm/kg and a pH of 7.7. Ten lenses were stored and evaluated as described in Examples 3-7. Superficial punctuate staining was observed in one of the eight eyes (12.5%) which were screened.

## Claims

1. The use of a solution having an osmolality of 220 mOsm/kg or greater and a conductivity of 4 mS/cm or greater for contacting an uncoated ophthalmic lens to reduce the occurrence of superficial punctate staining on the cornea resulting from wearing said lens, wherein said lens comprises polymeric units derived from at least one silicone containing component.

2. The use of claim 1 wherein said solution has an osmolality of 230 mOsm/kg or greater.

3. The use of claim 1 wherein said solution has a conductivity of 5 mS/cm or greater.

4. The use of claim 1 wherein said solution comprises at least one salt, non-ionic compound or a mixture thereof.

5. The use of claim 4 wherein said salt is selected from the group consisting of sodium chloride, sodium sulfate, sodium acetate, sodium citrate, sodium borate, sodium phosphate, sodium lactate, sodium hydrogenphosphate, sodium dihydrogenphosphate, potassium chloride, potassium sulfate, potassium acetate, potassium citrate, potassium borate, potassium phosphate, potassium hydrogenphosphate, potassium dihydrogenphosphate, calcium chloride, calcium sulfate, calcium acetate, calcium citrate, calcium borate, calcium phosphate, calcium lactate, calcium hydrogenphosphate, calcium dihydrogenphosphate, magnesium chloride, magnesium sulfate, magnesium acetate, magnesium citrate, magnesium borate, magnesium phosphate, magnesium lactate, magnesium hydrogenphosphate, magnesium dihydrogenphosphate and mixtures thereof.

6. The use of claim 4 wherein said non-ionic compound is selected from the group consisting of polyethylene glycols, polyhydroxy compounds, polyether compounds, sugars, polyvinylamides, dextrans, cyclodextrans and mixtures thereof.

7. The use of claim 4 wherein said salt is selected from the group consisting of sodium chloride, sodium sulfate, sodium borate and mixtures thereof.

8. The use of claim 1, 5 or 6 wherein said solution comprises saline solutions, buffered solutions and buffered saline solutions.

9. The use of claim 6, wherein said salt is present in an amount between 0.01 to 5.0 weight %

10. The use of claim 6, wherein said salt is present in an amount between 0.1 to 3.0 weight %

11. The use of claim 1 wherein said ophthalmic lens further comprises at least one polymeric wetting agent.

12. The use of claim 11 wherein said polymeric internal wetting agent is selected from the group consisting of polyamides, polylactones, polyimides, polylactams and functionalized polyamides, polylactones, polyimides, polylactams, hydrophilic prepolymers and mixtures thereof.

13. The use of claim 11 wherein said polymeric internal wetting agent is selected from the group consisting of homopolymers and copolymers comprising N-vinylpyrrolidone, N-vinyl-N-methylacetamide, (meth)acrylic acid, N,N-dimethylacrylamide and mixtures thereof.

14. The use of claim 1 wherein said lens further comprises at least one antimicrobial metal salt.

15. The use of claim 1 wherein said solution is a packaging solution.

16. The use of claim 1 wherein said solution is a storage solution.

17. The use of claim 1 wherein said solution is a cleaning solution.

18. The use of claim 1 wherein said solution is contained within a package comprising a reservoir, and said lens immersed in said solution.

19. A method for reducing the occurrence of superficial punctate staining comprising the step of contacting an uncoated ophthalmic lens comprising polymer units derived from at least one silicone containing component in a solution having an osmolality of 220 mOsm/kg or greater and a conductivity of 4 mS/cm or greater.

20. The method of claim 19 wherein the occurrence of superficial punctate staining is less than 50%.

21. The method of claim 19 wherein the occurrence of superficial punctate staining is less than 30%.

22. The method of claim 19 wherein the occurrence of superficial punctate staining is less than 10%.

23. The method of claim 19 wherein said solution has an osmolality of 230 mOsm/kg or greater.

24. The method of claim 19 wherein said solution has a conductivity of 5 mS/cm or greater

25. The method of claim 19 wherein said solution comprises at least one salt, non-ionic compound or a mixture thereof.

26. The method of claim 25 wherein said salt is selected from the group consisting of sodium chloride, sodium sulfate, sodium acetate, sodium citrate, sodium borate, sodium phosphate, sodium lactate, sodium hydrogenphosphate, sodium dihydrogenphosphate, potassium chloride, potassium sulfate, potassium acetate, potassium citrate, potassium borate, potassium phosphate, potassium hydrogenphosphate, potassium dihydrogenphosphate, calcium chloride, calcium sulfate, calcium acetate, calcium citrate, calcium borate, calcium phosphate, calcium lactate, calcium hydrogenphosphate, calcium dihydrogenphosphate, magnesium chloride, magnesium sulfate, magnesium acetate, magnesium citrate, magnesium borate, magnesium phosphate, magnesium lactate, magnesium hydrogenphosphate, magnesium dihydrogenphosphate and mixtures thereof.

27. The method of claim 25 wherein said salt is selected from the group consisting of sodium chloride, sodium sulfate, sodium borate and mixtures thereof.

28. The method of claim 25 wherein said non-ionic compound is selected from the group consisting of polyethylene glycols, polyhydroxy compounds, polyether compounds, sugars, polyvinylamides, dextrans, cyclodextrans and mixtures thereof.

29. The method of claim 25, wherein said salt is present in an amount between 0.01 to 5.0 weight %

30. The method of claim 19 wherein said solution comprises saline solutions, buffered solutions and buffered saline solutions.

31. The method of claim 25, wherein said salt is present in an amount between 0.1 to 3.0 weight %

32. The method of claim 19 where said ophthalmic lens further comprises at least one polymeric wetting agent.

33. The method of claim 32 wherein said polymeric internal wetting agent is selected from the group consisting of polyamides, polylactones, polyimides, polylactams and functionalized polyamides, polylactones, polyimides, polylactams, hydrophilic Prepolymers and mixtures thereof.

34. The method of claim 32 wherein said polymeric internal wetting agent is selected from the group consisting of homopolymers and copolymers comprising N-vinylpyrrolidone, N-vinyl-N-methylacetamide, (meth)acrylic acid, N,N-dimethylacrylamide and mixtures thereof.

35. The method of claim 19 wherein said lens further comprises at least one antimicrobial metal salt.

36. The method of claim 19 wherein said solution is a packaging solution.

## Patentansprüche

1. Verwendung einer Lösung mit einer Osmolalität von 220 mOsm/kg oder mehr und einer Leitfähigkeit von 4 mS/cm oder mehr zum Inkontaktbringen mit einer nicht-beschichteten augenoptischen Linse, um das Auftreten von oberflächlicher punktförmiger Einfärbung auf der Hornhaut zu verringern, die vom Tragen der Linse herrührt, wobei die Linse Polymereinheiten umfasst, die von wenigstens einer silikonhaltigen Komponente abgeleitet sind.

2. Verwendung nach Anspruch 1, wobei die Lösung eine Osmolalität von 230 mOsm/kg oder mehr besitzt.

3. Verwendung nach Anspruch 1, wobei die Lösung eine Leitfähigkeit von 5 mS/cm oder mehr besitzt.

4. Verwendung nach Anspruch 1, wobei die Lösung wenigstens ein Salz, eine nicht-ionische Verbindung oder eine Mischung davon umfasst.

5. Verwendung nach Anspruch 4, wobei das Salz ausgewählt ist aus der Gruppe, bestehend aus Natriumchlorid, Natriumsulfat, Natriumacetat, Natriumcitrat, Natriumborat, Natriumphosphat, Natriumlactat, Natriumhydrogenphosphat, Natriumdihydrogenphosphat, Kaliumchlorid, Kaliumsulfat, Kaliumacetat, Kaliumcitrat, Kaliumborat, Kaliumphosphat, Kaliumhydrogenphosphat, Kaliumdihydrogenphosphat, Calciumchlorid, Calciumsulfat, Calciumacetat,
Calciumcitrat, Calciumborat, Calciumphosphat, Calciumlactat, Calciumhydrogenphosphat, Calciumdihydrogenphosphat, Magnesiumchlorid, Magnesiumsulfat, Magnesiumacetat, Magnesiumcitrat, Magnesiumborat, Magnesiumphosphat, Magnesiumlactat, Magnesiumhydrogenphosphat, Magnesiumdihydrogenphosphat und Mischungen davon.

6. Verwendung nach Anspruch 4, wobei die nicht-ionische Verbindung ausgewählt ist aus der Gruppe, bestehend aus Polyethylenglykolen, Polyhydroxyverbindungen, Polyetherverbindungen, Zuckern, Polyvinylamiden, Dextranen, Cyclodextranen und Mischungen davon.

7. Verwendung nach Anspruch 4, wobei das Salz ausgewählt ist aus der Gruppe, bestehend aus Natriumchlorid, Natriumsulfat, Natriumborat und Mischungen davon.

8. Verwendung nach Anspruch 1, 5 oder 6, wobei die Lösung Kochsalzlösungen, gepufferte Lösungen und gepufferte Kochsalzlösungen umfasst.

9. Verwendung nach Anspruch 6, wobei das Salz in einer Menge zwischen 0,01 und 5,0 Gew.-% vorliegt.

10. Verwendung nach Anspruch 6, wobei das Salz in einer Menge zwischen 0,1 bis 3,0 Gew.-% vorliegt.

11. Verwendung nach Anspruch 1, wobei die augenoptische Linse weiter wenigstens ein polymeres Benetzungsmittel umfasst.

12. Verwendung nach Anspruch 11, wobei das polymere interne Benetzungsmittel ausgewählt ist aus der Gruppe, bestehend aus Polyamiden, Polylactonen, Polyimiden, Polylactamen und funktionalisierten Polyamiden, Polylactonen, Polyimiden, Polylactamen, hydrophilen Präpolymeren und Mischungen davon.

13. Verwendung nach Anspruch 11, wobei das polymere interne Benetzungsmittel ausgewählt ist aus der Gruppe, bestehend aus Homopolymeren und Copolymeren, die N-Vinylpyrrolidon, N-Vinyl-N-methylacetamid, (Meth)acrylsäure, N,N-Dimethylacrylamid und Mischungen davon umfassen.

14. Verwendung nach Anspruch 1, wobei die Linse weiter wenigstens ein antimikrobielles Metallsalz umfasst.

15. Verwendung nach Anspruch 1, wobei die Lösung eine Verpackungslösung ist.

16. Verwendung nach Anspruch 1, wobei die Lösung eine Aufbewahrungslösung ist.

17. Verwendung nach Anspruch 1, wobei die Lösung eine Reinigungslösung ist.

18. Verwendung nach Anspruch 1, wobei die Lösung in einer Verpackung enthalten ist, die ein Reservoir umfasst, und die Linse in der Lösung eingetaucht ist.

19. Verfahren zur Verringerung des Auftretens von oberflächlicher punktförmiger Einfärbung, das den Schritt des Inkontaktbringens einer nicht-beschichteten augenoptischen Linse, die Polymereinheiten umfasst, die von wenigstens einer silikonhaltigen Komponente abgeleitet sind, in einer Lösung mit einer Osmolalität von 220 mOsm/kg oder mehr und einer Leitfähigkeit von 4 mS/cm oder mehr umfasst.

20. Verfahren nach Anspruch 19, wobei das Auftreten von oberflächlicher punktförmiger Einfärbung weniger als 50% beträgt.

21. Verfahren nach Anspruch 19, wobei das Auftreten von oberflächlicher punktförmiger Einfärbung weniger als 30% beträgt.

22. Verfahren nach Anspruch 19, wobei das Auftreten von oberflächlicher punktförmiger Einfärbung weniger als 10% beträgt.

23. Verfahren nach Anspruch 19, wobei die Lösung eine Osmolalität von 230 mOsm/kg oder mehr besitzt.

24. Verfahren nach Anspruch 19, wobei die Lösung eine Leitfähigkeit von 5 mS/cm oder mehr besitzt.

25. Verfahren nach Anspruch 19, wobei die Lösung wenigstens ein Salz, eine nicht-ionische Verbindung oder eine Mischung davon umfasst.

26. Verfahren nach Anspruch 25, wobei das Salz ausgewählt ist aus der Gruppe, bestehend aus Natriumchlorid, Natriumsulfat, Natriumacetat, Natriumcitrat, Natriumborat, Natriumphosphat, Natriumlactat, Natriumhydrogenphosphat, Natriumdihydrogenphosphat, Kaliumchlorid, Kaliumsulfat, Kaliumacetat, Kaliumcitrat, Kaliumborat, Kaliumphosphat, Kaliumhydrogenphosphat, Kaliumdihydrogenphosphat, Calciumchlorid, Calciumsulfat, Calciumacetat, Calciumcitrat, Calciumborat, Calciumphosphat, Calciumlactat, Calciumhydrogenphosphat, Calciumdihydrogenphosphat, Magnesiumchlorid, Magnesiumsulfat, Magnesiumacetat, Magnesiumcitrat, Magnesiumborat, Magnesiumphosphat, Magnesiumlactat, Magnesiumhydrogenphosphat, Magnesiumdihydrogenphosphat und Mischungen davon.

27. Verfahren nach Anspruch 25, wobei das Salz ausgewählt ist aus der Gruppe, bestehend aus Natriumchlorid, Natriumsulfat, Natriumborat und Mischungen davon.

28. Verfahren nach Anspruch 25, wobei die nicht-ionische Verbindung ausgewählt ist aus der Gruppe, bestehend aus Polyethylenglykolen, Polyhydroxyverbindungen, Polyetherverbindungen, Zuckern, Polyvinylamiden, Dextranen, Cyclodextranen und Mischungen davon.

29. Verfahren nach Anspruch 25, wobei das Salz in einer Menge zwischen 0,01 und 5,0 Gew.-% vorliegt.

30. Verfahren nach Anspruch 19, wobei die Lösung Kochsalzlösungen, gepufferte Lösungen und gepufferte Kochsalzlösungen umfasst.

31. Verfahren nach Anspruch 25, wobei das Salz in einer Menge zwischen 0,1 bis 3,0 Gew.-% vorliegt.

32. Verfahren nach Anspruch 19, wobei die augenoptische Linse weiter wenigstens ein polymeres Benetzungsmittel umfasst.

33. Verfahren nach Anspruch 32, wobei das polymere interne Benetzungsmittel ausgewählt ist aus der Gruppe, bestehend aus Polyamiden, Polylactonen, Polyimiden, Polylactamen und funktionalisierten Polyamiden, Polylactonen, Polyimiden, Polylactamen, hydrophilen Präpolymeren und Mischungen davon.

34. Verfahren nach Anspruch 32, wobei das polymere interne Benetzungsmittel ausgewählt ist aus der Gruppe, bestehend aus Homopolymeren und Copolymeren, die N-Vinylpyrrolidon, N-Vinyl-N-methylacetamid, (Meth)acrylsäure, N,N-Dimethylacrylamid und Mischungen davon umfassen.

35. Verfahren nach Anspruch 19, wobei die Linse weiter wenigstens ein antimikrobielles Metallsalz umfasst.

36. Verfahren nach Anspruch 19, wobei die Lösung eine Verpackungslösung ist.

## Revendications

1. Utilisation d'une solution ayant une osmolalité de 220 mOsm/kg ou plus et une conductivité de 4 mS/cm ou plus destinée à être mise en contact avec une lentille de contact non revêtue pour réduire l'occurrence d'un piqueté cornéen superficiel résultant du port de ladite lentille, dans laquelle ladite lentille comprend des motifs polymères dérivés d'au moins un composant à base de silicone.

2. Utilisation selon la revendication 1, dans laquelle ladite solution a une osmolalité de 230 mOsm/kg ou plus.

3. Utilisation selon la revendication 1, dans laquelle ladite solution a une conductivité de 5 mS/cm ou plus.

4. Utilisation selon la revendication 1, dans laquelle ladite solution comprend au moins un sel, un composé non ionique ou un mélange de ceux-ci.

5. Utilisation selon la revendication 4, dans laquelle ledit sel est choisi dans le groupe constitué par le chlorure de sodium, le sulfate de sodium, l'acétate de sodium, le citrate de sodium, le borate de sodium, le phosphate de sodium, le lactate de sodium, l'hydrogéno-phosphate de sodium, le dihydrogéno-phosphate de sodium, le chlorure de potassium, le sulfate de potassium, l'acétate de potassium, le citrate de potassium, le borate de potassium, le phosphate de potassium, l'hydrogénophosphate de potassium, le dihydrogénophosphate de potassium, le chlorure de calcium, le sulfate de calcium, l'acétate de calcium, le citrate de calcium, le borate de calcium, le phosphate de calcium, le lactate de calcium, l'hydrogénophosphate de calcium, le dihydrogénophosphate de calcium, le chlorure de magnésium, le sulfate de magnésium, l'acétate de magnésium, le citrate de magnésium, le borate de magnésium, le phosphate de magnésium, le lactate de magnésium, l'hydrogénophosphate de magnésium, le dihydrogénophosphate de magnésium et leurs mélanges.

6. Utilisation selon la revendication 4, dans laquelle ledit composé non ionique est choisi dans le groupe constitué par les polyéthylène glycols, les composés polyhydroxy, les composés polyéther, les sucres, les polyvinylamides, les dextranes, les cyclodextranes et leurs mélanges.

7. Utilisation selon la revendication 4, dans laquelle ledit sel est choisi dans le groupe constitué par le chlorure de sodium, le sulfate de sodium, le borate de sodium et leurs mélanges.

8. Utilisation selon les revendications 1, 5 ou 6, dans laquelle ladite solution comprend les solutions salines, les solutions tamponnées et les solutions salines tamponnées.

9. Utilisation selon la revendication 6, dans laquelle ledit sel est présent en une quantité comprise entre 0,01 et 5,0 % en poids.

10. Utilisation selon la revendication 6, dans laquelle ledit sel est présent en une quantité comprise entre 0,1 et 3,0 % en poids.

11. Utilisation selon la revendication 1, dans laquelle ladite lentille de contact comprend, en outre, au moins un agent de mouillage polymère.

12. Utilisation selon la revendication 11, dans laquelle ledit agent de mouillage polymère interne est choisi dans le groupe constitué par les polyamides, les polylactones, les polyimides, les polylactames et les polyamides, polylactones, polyimides, polylactames fonctionnalisés, les prépolymères hydrophiles et leurs mélanges.

13. Utilisation selon la revendication 11, dans laquelle ledit agent de mouillage polymère interne est choisi dans le groupe constitué par les homopolymères et les copolymères comprenant la N-vinylpyrrolidone, le N-vinyl-N-méthylacétamide, l'acide (méth)acrylique, le N,N-diméthylacrylamide et leurs mélanges.

14. Utilisation selon la revendication 1, dans laquelle ladite lentille comprend, en outre, au moins un sel métallique antimicrobien.

15. Utilisation selon la revendication 1, dans laquelle ladite solution est une solution de conditionnement.

16. Utilisation selon la revendication 1, dans laquelle ladite solution est une solution de conservation.

17. Utilisation selon la revendication 1, dans laquelle ladite solution est une solution de nettoyage.

18. Utilisation selon la revendication 1, dans laquelle ladite solution est contenue dans un étui comprenant un réservoir, et ladite lentille est immergée dans ladite solution.

19. Procédé pour réduire l'occurrence d'un piqueté cornéen superficiel comprenant l'étape consistant à mettre en contact une lentille de contact non revêtue comprenant des motifs polymères dérivés d'au moins un composant à base de silicone avec une solution ayant une osmolalité de 220 mOsm/kg ou plus et une conductivité de 4 mS/cm ou plus.

20. Procédé selon la revendication 19, dans lequel l'occurrence du piqueté cornéen superficiel est inférieure à 50 %.

21. Procédé selon la revendication 19, dans lequel l'occurrence du piqueté cornéen superficiel est inférieure à 30 %.

22. Procédé selon la revendication 19, dans lequel l'occurrence du piqueté cornéen superficiel est inférieure à 10 %.

23. Procédé selon la revendication 19, dans lequel ladite solution a une osmolalité de 230 mOsn/kg ou plus.

24. Procédé selon la revendication 19, dans lequel ladite solution a une conductivité de 5 mS/cm ou plus.

25. Procédé selon la revendication 19, dans lequel ladite solution comprend au moins un sel, un composé non ionique ou un mélange de ceux-ci.

26. Procédé selon la revendication 25, dans lequel ledit sel est choisi dans le groupe constitué par le chlorure de sodium, le sulfate de sodium, l'acétate de sodium, le citrate de sodium, le borate de sodium, le phosphate de sodium, le lactate de sodium, l'hydrogéno-phosphate de sodium, le dihydrogéno-phosphate de sodium, le chlorure de potassium, le sulfate de potassium, l'acétate de potassium, le citrate de potassium, le borate de potassium, le phosphate de potassium, l'hydrogénophosphate de potassium, le dihydrogénophosphate de potassium, le chlorure de calcium, le sulfate de calcium, l'acétate de calcium, le citrate de calcium, le borate de calcium, le phosphate de calcium, le lactate de calcium, l'hydrogénophosphate de calcium, le dihydrogénophosphate de calcium, le chlorure de magnésium, le sulfate de magnésium, l'acétate de magnésium, le citrate de magnésium, le borate de magnésium, le phosphate de magnésium, le lactate de magnésium, l'hydrogénophosphate de magnésium, le dihydrogénophosphate de magnésium et leurs mélanges.

27. Procédé selon la revendication 25, dans lequel ledit sel est choisi dans le groupe constitué par le chlorure de sodium, le sulfate de sodium, le borate de sodium et leurs mélanges.

28. Procédé selon la revendication 25, dans lequel ledit composé non ionique est choisi dans le groupe constitué par les polyéthylènes glycols, les composés polyhydroxy, les composés polyéther, les sucres, les polyvinylamides, les dextranes, les cyclodextranes et leurs mélanges.

29. Procédé selon la revendication 25, dans lequel ledit sel est présent en une quantité comprise entre 0,01 et 5,0 % en poids.

30. Procédé selon la revendication 19, dans lequel ladite solution comprend les solutions salines, les solutions tamponnées et les solutions salines tamponnées.

31. Procédé selon la revendication 25, dans lequel ledit sel est présent en une quantité comprise entre 0,1 et 3,0 % en poids.

32. Procédé selon la revendication 19, dans lequel ladite lentille de contact comprend, en outre, au moins un agent de mouillage polymère.

33. Procédé selon la revendication 32, dans lequel ledit agent de mouillage polymère interne est choisi dans le groupe constitué par les polyamides, les polylactones, les polyimides, les polylactames et les polyamides, polylactones, polyimides, polylactames fonctionnalisés, les prépolymères hydrophiles et leurs mélanges.

34. Procédé selon la revendication 32, dans lequel ledit agent de mouillage polymère interne est choisi dans le groupe constitué par les homopolymères et les copolymères comprenant la N-vinylpyrrolidone, le N-vinyl-N-méthylacétamide, l'acide (méth)acrylique, le N,N-diméthylacrylamide et leurs mélanges.

35. Procédé selon la revendication 19, dans lequel ladite lentille comprend, en outre, au moins un sel métallique antimicrobien.

36. Procédé selon la revendication 19, dans lequel ladite solution est une solution de conditionnement.
